(19) Europäisches Patentamt
European Patent Office
Office européen des brevets

(11) Numéro de publication: **0 030 500**
**B1**

(12) **FASCICULE DE BREVET EUROPEEN**

(45) Date de publication du fascicule du brevet: **27.07.83**

(21) Numéro de dépôt: **80401718.4**

(22) Date de dépôt: **02.12.80**

(51) Int. Cl.³: **B 01 F 5/06,** B 01 J 14/00, B 01 J 19/24, C 07 D 263/44

(54) **Appareillage pour la réalisation de réactions chimiques et procédé utilisant ledit appareillage.**

(30) Priorité: **05.12.79 FR 7929898**

(43) Date de publication de la demande:
**17.06.81 Bulletin 81/24**

(45) Mention de la délivrance du brevet:
**27.07.83 Bulletin 83/30**

(84) Etats contractants désignés:
**AT BE CH DE FR GB IT LI LU NL SE**

(56) Documents cités:
**DE - B - 2 130 134**
**FR - A - 887 038**
**FR - A - 1 502 281**
**FR - A - 2 046 362**
**FR - A - 2 073 356**
**FR - A - 2 383 887**
**US - A - 4 222 671**

(73) Titulaire: **Gignier, Jean**
**4, rue des Capucines**
**F-92190 Meudon (FR)**

(72) Inventeur: **Gignier, Jean**
**4, rue des Capucines**
**F-92190 Meudon (FR)**

(74) Mandataire: **Combe, André et al,**
**CABINET BEAU DE LOMENIE 55 rue d'Amsterdam**
**F-75008 Paris (FR)**

**EP 0 030 500 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Appareillage pour la réalisation de réactions chimiques et procédé utilisant ledit appareillage

La présente invention concerne un appareillage pour la réalisation de réactions chimiques et un procédé utilisant ledit appareillage.

Les réactions chimiques mettent en jeu simultanément transferts de matières et transferts de chaleur et les appareillages où elles sont effectuées doivent donc réaliser simultanément les effets suivants:

— bonne capacité d'échange de chaleur,

— turbulence et cisaillement, déjà utiles pour assurer un mélange intime dans le cas d'une phase unique liquide ou gazeuse, et d'autant plus nécessaires qu'on se trouve en présence de deux phases (liquide-solide, liquide-liquide, ou liquide-gaz) ou même de trois phases (liquide-solide-gaz).

La bonne réalisation simultanée de ces divers effets est plus particulièrement difficile en continu, où l'on n'a pas la faculté d'introduire plus ou moins lentement l'un des réactifs pour s'adapter aux limitations de l'appareillage.

Les formes classiques d'appareillage: réacteurs agités d'une part, et réacteurs tubulaires de l'autre, ont toutes deux des limitations.

Les réacteurs agités sont limités sur le plan des échanges de chaleur. D'autre part, l'agitateur qui y réalise la turbulence nécessaire comporte obligatoirement une garniture d'étanchéité autour de son arbre qui pose un sérieux problème technologique à haute pression.

Les réacteurs tubulaires sont excellents pour réaliser un écoulement piston donnant le plus petit volume réactionnel possible et une grande capacité d'échange de chaleur. Mais la réalisation d'une forte turbulence nécessite une vitesse importante d'où une section réduite, ce que augmente proportionnellement la longueur pour maintenir le même volume réactionnel.

Il a été proposé pour la réalisation, en continu, de réactions aux interfaces entre au moins deux phases non miscibles entre elles, un réacteur sous forme de tube caractérisé en ce qu'il comprend une suite alternée de sections de séjour ayant un diamètre intérieur de tube plus élevé et de sections mélangeuses ayant un diamètre intérieur de section plus faible, ces dernières sections assurant des nombres de Reynolds supérieurs à 2000. Les réalisations matérielles d'un réacteur de ce type décrites dans le brevet français 70 14 671 consistent en une série de tubes en ligne, ou en une série de tubes en forme de serpentins, ou en des douilles interchangeables. De telles réalisations sont techniquement relativement difficiles.

La présente invention vise tout d'abord un appareillage pratique pour la réalisation de réactions chimiques en milieu hétérogène mettant en jeu simultanément des transferts de chaleur et des transferts de matière.

L'invention concerne donc un réacteur pour la réalisation en continu de réactions chimiques hétérogènes impliquant des échanges thermiques et des échanges de matière, ledit réacteur étant formé d'une suite alternée de sections de séjour constituées d'un faisceau de tube de séjour (1), paralléles, et de sections melangeuses, caractérisé en ce que les tubes de séjour (1) ont leurs extrémités fixées dans deux plaques (3) et sont plongés dans un fluide échangeur de chaleur, et en ce que deux têtes (4) fixées auxdites plaques comportent des alvéoles (2), reliant l'un à l'autre les tubes (1) du faisceau, chaque alvéole étant aménagée de façon à provoquer une perte de charge de 50 à 500 fois plus forte que la perte de charge dans la partie de séjour correspondante, et en ce que lesdits tubes de séjour présentent une surface globable d'échange de chaleur telle que le rapport entre cette surface exprimée en mètres carrés et le volume intérieur des tubes exprimé en mètres cubes est compris entre 12 et 500.

Les figures 1 à 7 illustrent diverses réalisations de l'invention.

La figure 1 représente en coupe l'appareil selon l'invention; cet appareil comporte un faisceau de tubes de séjour 1 baignant dans un liquide thermostatique chargé d'apporter ou d'enlever les calories de la réaction qui se déroule dans lesdits tubes; les extrémités de ces tubes sont fixées dans deux plaques 3 qui constituent d'ailleurs, avec une paroi latérale convenable, l'enceinte dans laquelle se trouve le liquide thermostatique; sur ces plaques 3 on fixe des têtes 4 dans lesquelles sont ménagés, par tout moyen connu tel qu'emboutissage ou usinage, des alvéoles tels que 2 qui réunissent les extrémités de tubes deux par deux.

Ces alvéoles doivent bien évidemment être convenablement positionnés pour pouvoir relier effectivement les extrémités des deux tubes. Mais, de plus, leur dimension (diamètre par exemple) et leur forme doivent être telles qu'ils jouent le rôle de mélangeurs énergiques pour le fluide (liquide) qui les traverse.

Sur les figures 2 à 7, on a représenté divers modes de réalisation de ces alvéoles mélangeurs; les figures 3, 5 et 7 sont des coupes respectives des alvéoles selon les figures 2, 4 et 6. Ces diverses formes d'alvéoles permettent de résoudre la plupart des problèmes posés par les réactions chimiques que l'on réalise dans l'appareil considéré.

Bien que les caractéristiques précises de chacune des parties (tubes de séjour et alvéoles) de l'appareil selon l'invention dépendent de la réaction qui doit être réalisée dans cet appareil, il apparaît généralement:

— qui la partie alvéole de l'appareil doit être aménagée de façon qu'elle provoque une perte

de charge de 50 à 500 fois plus forte que la perte de charge dans la partie séjour de l'appareil,

— que, compte tenu de la nécessité de réaliser des échanges thermiques au niveau des tubes de séjour dans l'appareil selon l'invention, le rapport de la surface globale d'échange de chaleur, exprimée en mètres carrés, des parties tubulaires, au volume réactionnel total, exprimé en mètres cubes, doit être compris entre 12 et 500 et, de préférence, entre 100 et 400.

Une autre manière de réaliser l'ensemble de conduits tubulaires peut être avantageuse pour des matériaux de construction non métalliques. Alors que les tubes réunis en faisceau décrits et représentés à la figure 1 sont la réalisation la plus pratique dans le cas de métaux, d'autres matériaux, tels que le graphite largement utilisé dans la chimie, sont fréquemment oeuvrés d'une manière différente pour tirer le meilleur parti de leurs propriétés mécaniques et thermiques. La matière se présentant initialement sous forme de blocs, les conduits tubulaires sont réalisés en forant des trous allant d'un bout à l'autre du bloc. Les alvéoles déjà décrits sont placés sur les extrémités de ces conduits tubulaires réunissant les extrémités de deux conduits adjacents, comme représenté à la figure 1 et réalisent comme précédemment une seule conduite de grande longueur allant de l'entrée à la sortie de l'appareil. Il est possible de ménager pour le fluide caloporteur des passages également percés dans la masse du bloc, ne rencontrant pas les conduits du fluide de procédé, mais permettant d'échanger la chaleur avec eux grâce à la bonne conductivité thermique du graphite.

Grâce à ces possibilités, il est notamment possible de réaliser dans l'appareil selon l'invention des réactions chimiques se déroulant en phase hétérogène entre une suspension solide dispersée dans un milieu réactionnel et un gaz dissous dans ledit milieu. Dans ce cas, pour que le gaz soit maintenu en solution à la température choisie pour la réaction, il convient souvent d'opérer sous des pressions élevées et l'appareillage selon l'invention est particulièrement intéressant pour de telles réactions.

On a trouvé que l'appareillage selon l'invention était spécialement adapté à la réaction, au sein d'un milieu liquide, d'une suspension d'un acide amine tel que, par exemple, et, de préférence, la phénylglycine ou la parahydroxyphénylglycine avec du phosgène qui se trouve en solution dans le milieu liquide. Une telle réaction se déroule avec un excès de phosgène, cet excès étant obtenu par la dissolution d'une quantité convenable de phosgène dans le milieu réactionnel compte tenu de la quantité d'aminoacide présent dans ledit milieu. Sitôt le mélange réactionnel sorti du réacteur, on peut libérer le phosgène encore présent dans le milieu par un simple abaissement de la pression et détente avec vaporisation du réactif. L'excès de réactif est ainsi éliminé très rapidement, ce qui est généralement favorable au rendement global de la réaction.

On décrit ci-après les conditions que l'on peut utiliser pour réaliser la réaction de la parahydroxyphénylglycine avec le phosgène en utilisant un appareillage selon l'invention.

Exemple

La parahydroxyphénylglycine est un acide aminé dont le chlorure d'acide (chlorhydraté sur le groupe $NH_2$) est un intermédiaire utilisé dans la production d'antibiotiques semi-synthétiques. La première étape de cette fabrication, décrite dans le brevet française n° 2 267 307 et dans d'autres brevets correspondants tels que le brevet des Etats-Unis d'Amérique n° 3 925 418, est une phosgénation réalisant la réaction ci-dessous.

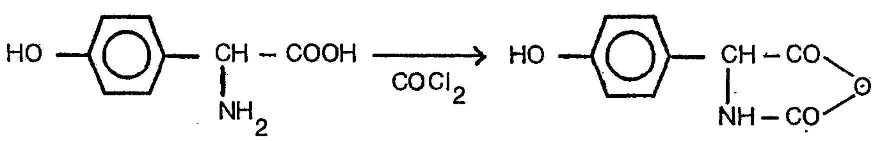


(1) p-hydroxyphenylglycine

(2) anhydride de Leuch

Cette réaction est couramment réalisée dans un réacteur agité. On introduit un excès de phosgène de 1, 6 à 2 fois la quantité stoechiométrique et on porte le contenu du réacteur à 65°C. Le temps nécessaire entre la fin de l'introduction du phosgène et la terminaison de la réaction (matérialisée par la disparition du réactif (1) de l'équation ci-dessus qui est à l'état solide en suspension dans le milieu avant sa transformation en (2)) est de l'ordre de 45 à 70 min, et il est de 4 à 6 h pour éliminer complètement l'excès de phosgène. Le fluide chauffant est de la vapeur basse pression donnant une température de paroi intérieure du réacteur supérieure à 100°C, ce qui amène des dégradations du produit.

Avec un appareil du type décrit ici, équipé de tubes de diamètre 16 mm, avec des chambres de raccordement représentées sur les figures 2 et 3, l'opération depuis le mélange du phosgène jusqu'à l'élimination complète de l'excès de phosgène prend moins de 30 min, le fluide chauffant étant seulement à 65—70°C.

**Revendications**

1. Réacteur pour la réalisation en continu de réactions chimiques hétérogènes impliquant des échanges thermiques et des échanges de matière, ledit réacteur étant formé d'une suite alternée de sections de séjour constituées d'un faisceau de tubes de séjour (1), paralleles, et de sections mélangeuses, caractérisé en ce que les tubes de séjour (1) ont leurs extrémités fixées dans deux plaques (3) et sont plongés dans un fluide échangeur de chaleur, et en ce que deux têtes (4) fixées auxdites plaques comportent des alvéoles (2), reliant l'un à l'autre les tubes (1) du faisceau, chaque alvéole étant aménagée de façon à provoquer une perte de charge de 50 à 500 fois plus forte que la perte de charge dans la partie de séjour correspondante, et en ce que lesdits tubes de séjour présentent une surface globale d'échange de chaleur telle que le rapport entre cette surface exprimée en mètres carrés et le volume intérieur des tubes exprimé en mètres cubes est compris entre 12 et 500.

2. Procédé pour la réalisation de la réaction de phosgénation d'un aminoacide de façon à obtenir l'anhydride de Leuch, caractérisè en ce que ladite phosgénation est réalisée dans un milieu réactionnel dans lequel l'aminoacide est en suspension et le phosgène en solution et que l'on utilise un réacteur selon la revendication 1.

3. Procédé selon la revendication 2, caractérisé en ce que l'aminoacide est choisi parmi la phénylglycine et la parahydroxyphénylglycine à une température de 65—70°C.

**Claims**

1. Reactor for continuously producing heterogeneous chemical reactions implying heat exchanges and material exchanges, the said reactor being made up of an alternate succession of dwelling sections, constituted by a bundle of parallel dwelling tubes (1), and of mixing sections, characterised in that the dwelling tubes (1) have their ends secured in two plates (3) and are immersed inside a heat-exchanging fluid, and in that two heads (4) fixed on said plates, comprise alveoli (2) joining together the tubes (1) of the bundle, each alveolus being arranged so as to cause a loss of load 50 to 500 times stronger than the loss of load in the corresponding dwelling part, and in that the said dwelling tubes have a overall heat-exchanging surface such that the ration between said surface expressed in square metres and the inside volume of the tubes expressed in cubic metres is between 12 and 500.

2. Method for producing the phosgenation reaction of an aminoacid in order to obtain Leuch's anhydride, characterised in that the said phosgenation is produced in a reaction medium wherein the aminoacid is in suspension and the phosgene in solution and in that a reactor according to claim 1 is used.

3. Method according to claim 2, characterised in that the aminoacid is selected from phenylglycine and parahydroxyphenylglycine at a temperature of 65—70°C.

**Patentansprüche**

1. Reaktor zur kontinuierlichen Durchführung von heterogenen chemischen Reaktionen, bei denen Wärmeaustausch und Materialaustausch stattfinden, welcher Reaktor aus einer abwechselnden Folge von durch ein Bündel paralleler Rohre (1) gebildeten Verweilabschnitten und von Mischungsabschnitten gebildet ist, dadurch gekennzeichnet, daß die Verweilrohre (1) mit ihren Enden in zwei Platten (3) befestigt und in einem Wärmeaustauschermittel eingetaucht sind, und daß zwei auf den Platten befestigte Köpfe (4) die Rohre (1) des Bündels miteinander verbindende Ausnehmungen (2) aufweisen, wobei jede Ausnehmung derart angeordnet ist, daß sie einen 50 bis 500 mal stärkeren Chargenverlust als der Chargenverlust im entsprechenden Verweilteil bewirkt, und daß die Verweilrohre eine solche Wärmeaustauschgesamtoberfläche aufweisen, daß das Verhältnis zwischen dieser Oberfläche, ausgedrückt in Quadratmetern, und dem Innenvolumen der Rohre, ausgedrückt in Kubikmetern, zwischen 12 und 500 beträgt.

2. Verfahren zur Durchführung der Phosgenierungsreaktion einer Aminosäure, um das Leuchssche Anhydrid zu erhalten, dadurch gekennzeichnet, daß die Phosgenierung in einem Reaktionsmedium durchgeführt wird, in welchem die Aminosäure in Suspension und das Phosgen in Lösung sind und der Reaktor gemäß Anspruch 1 verwendet wird.

3. Verfahren nach Anspruch 2, dadurch gekennzeichnet, daß die Aminosäure von Phenylglycin und p-Hydroxyphenylglycin ausgewählt wird bei einer Temperatur von 65—70°C.

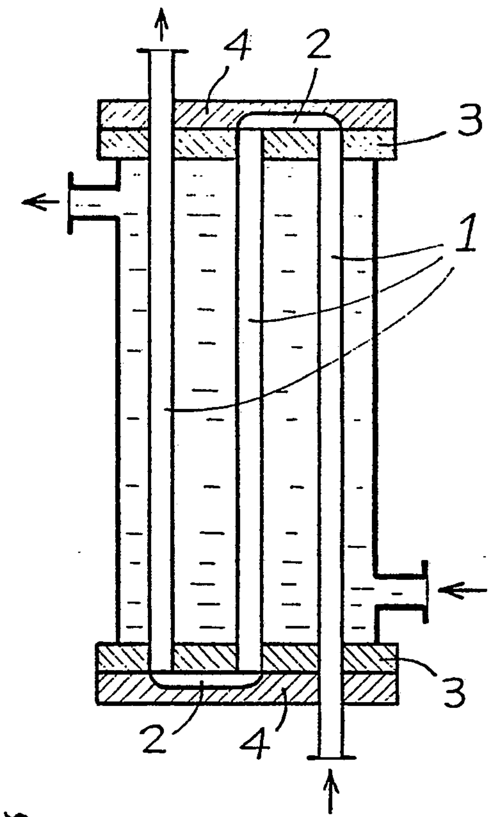


Fig-1

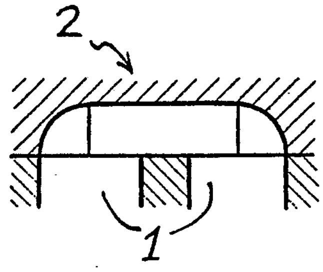


Fig-2

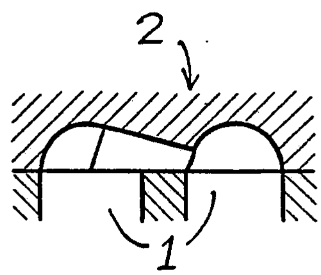


Fig-4

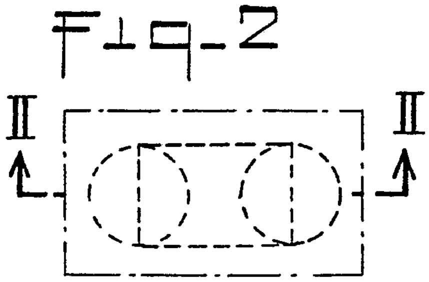


Fig-3

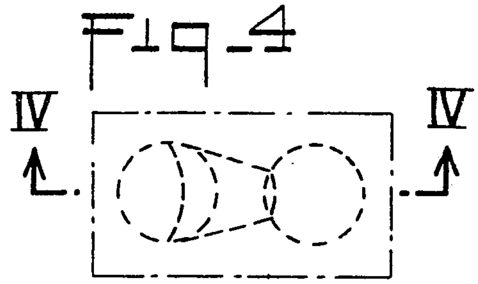


Fig-5

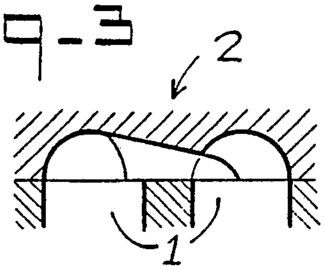


Fig-6

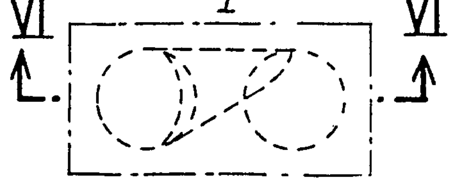


Fig-7